# EUROPEAN PATENT SPECIFICATION

(11) **EP 4 070 219 B1**
(45) Date of publication and mention of the grant of the patent: **06.05.2026**
(21) Application number: 20821392.6
(22) Date of filing: 02.12.2020
(51) Int. Cl.: G06F 21/62, H04W 12/02

(54) **NATURAL PSEUDONYMIZATION AND DOWNSTREAM PROCESSING**
NATÜRLICHE PSEUDONYMISIERUNG UND NACHFOLGENDE VERARBEITUNG
PSEUDONYMISATION NATURELLE ET TRAITEMENT EN AVAL

(30) Priority: 06.12.2019 US 201962945009 P
(43) Date of publication of application: 12.10.2022
(73) Proprietor: Solventum Intellectual Properties Company, Maplewood, MN 55144 (US)
(72) Inventor: YAROWSKY, David E., Saint Paul, Minnesota 55133-3427 (US); HAMBURGER, Marc, 8803 Zürich (CH); WEISER, Octavian, 41453 Neuss (DE)
(74) Representative: Vossius & Partner Patentanwälte Rechtsanwälte mbB
(86) International application number: PCT/IB2020/061376
(87) International publication number: WO 2021/111324

(56) References cited:
- GB-A- 2 561 241
- US-A1- 2014 280 261
- US-A1- 2016 063 269
- US-A1- 2018 096 102

## Description

### Technical Field

The present disclosure is related to systems and methods for data pseudonymization and obfuscation and using data pseudonymization and obfuscation.

### Brief Description of Drawings

The accompanying drawings are incorporated in and constitute a part of this specification and, together with the description, explain the advantages and principles of the invention. In the drawings,
Figure 1A illustrates a system diagram of one embodiment of a natural pseudonymization system 100; and Figures 1B-1E provide some example implementations of components illustrated in Figure 1A;
Figure 2A illustrates a flowchart of one embodiment of a natural pseudonymization system;
Figure 2B illustrates a flowchart to one example of a downstream processing system;
Figures 3A-3D illustrate various examples of how natural pseudonymization systems used for downstream processing; and
Figure 4A and 4B illustrates one example of input and output of a natural pseudonymization system.

In the drawings, like reference numerals indicate like elements. While the above-identified drawings, which may not be drawn to scale, set forth various embodiments of the present disclosure, other embodiments are also contemplated, as noted in the Detailed Description. In all cases, this disclosure describes the presently disclosed disclosure by way of representation of exemplary embodiments and not by express limitations. It should be understood that numerous other modifications and embodiments can be devised by those skilled in the art, which fall within the scope of this disclosure.

### Detailed Description

More and more sensitive and personal data is collected and used. Under various data law and regulations, data collection, processing, and storage require special care. For example, handling and exchanging documents with personal and sensitive health data are subject to data regulations, such as HIPPA ("Health Insurance Portability and Accountability Act") and GDPR ("General Data Protection Regulation"). In some cases, personal data is required to be deidentified. In some cases, sensitive data and documents that clinicians use for training, external or internal audits, testing module functionality or developing new features in hospital information systems needs pseudonymization. In some cases, secure storage of patient information in the cloud or hospital database needs pseudonymization and encryption of data. US 2016/063269 A1 discloses an outsourcing environment by which an outsourcing entity may delegate document-transformation tasks to at least one worker entity, while preventing the worker entity from gaining knowledge of sensitive items that may be contained within a non-obfuscated original document (NOD). The environment may transform the NOD into an obfuscated original document (OOD) by removing sensitive items from the NOD. The worker entity may perform formatting and/or other document-transformation tasks on the OOD, without gaining knowledge of the sensitive items in the NOD, to produce an obfuscated transformed document (OTD). The environment may then allow for the outsourcing entity to view a content-restored version of the OTD. US 2014/280261 A1 discloses a system which includes a software program capable of performing an aliasing function on the personally identifiable information ("PII") of a subject. The software can associate the alias with the PII and output the alias rather than the PII.

Various embodiments of the present disclosure are directed to natural pseudonymization. In some cases, deidentification of free text is done in English and various European languages (e.g., German, French, Flemish, English, Spanish, Italian) using natural pseudonymization techniques. Pseudonymization refers to the separation of data from direct identifiers (such as first name or social security number) so that linkage to the original identity is impossible to make without additional information. In some cases, a pseudonymization table is generated and stored separately on highly secure servers for real-time re-identification of patient documents. In some cases, the method includes natural pseudonymization, where sensitive or personal data is replaced by data with the same type, gender and language/region data. For example, a female name is replaced by another female name common in the local culture, or street Gritzenweg is replaced with Rosengasse, throughout the entire document, naturally preserving both context and the type of data.

**In** some embodiments, the method discerns between sensitive health and personal information and general or medical concepts needed for such clinical/medical text analysis applications including clinical decision support, medical study recruiting, clinical utilization management or medical coding by a mixture of word-context, phrase-context, word/phrase-internal, region-context and document-wide statistical models which effectively handling such natural language processing challenges such as complex whitespace, inter-word document markup, punctuation, nested expressions (e.g. Rue de Wilson) and compositional models (e.g. Carl-Schurz-Platz or Aktivierungsvorrichtung). In some cases, the method preserves local culture and meaning by utilizing careful same-type replacements enables identical output of document annotations for computer assisted coding systems. In some cases, the method enables identical assigned codes and identified evidence in examined texts to be generated in the original texts and their pseudonymized counterparts. In some cases, the method includes shifting dates relative to a randomly chosen baseline date, preserving time intervals needed, for example, for clinical, research and administrative inferences and decisions and introducing additional date-shift noise for dates increasingly far in the past.

The functions, algorithms, and methodologies described herein may be implemented in software in one embodiment. The software may consist of computer executable instructions stored on computer readable media or computer readable storage device such as one or more non-transitory memories or other type of hardware-based storage devices, either local or networked. Further, such functions correspond to modules or processors, which may be software, hardware, firmware or any combination thereof. Multiple functions may be performed in one or more modules or processors as desired, and the embodiments described are merely examples. The software may be executed on a digital signal processor, ASIC, microprocessor, or other type of processor operating on a computer system, such as a personal computer, server or other computer system, turning such computer system into a specifically programmed machine.

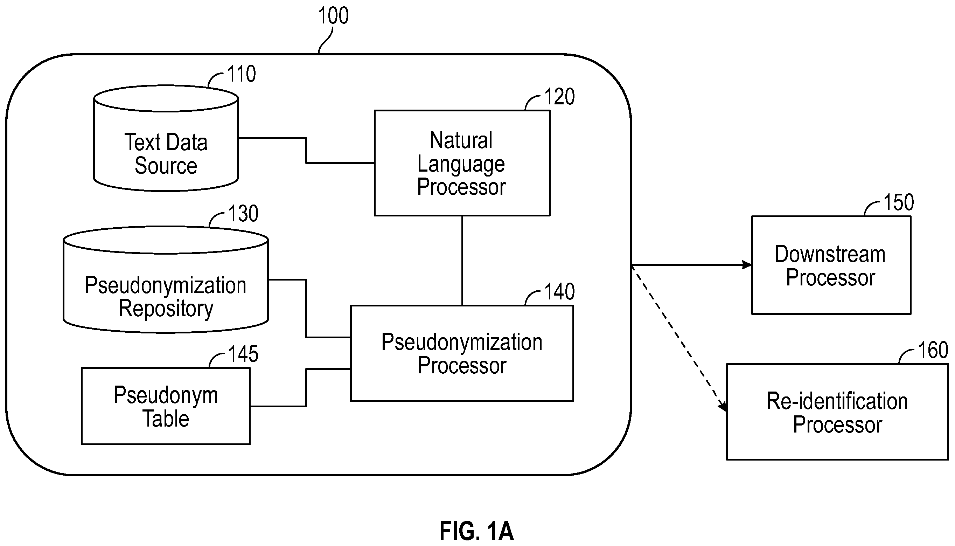

Figure 1A illustrates a system diagram of one embodiment of a natural pseudonymization system 100. In one example, the system 100 includes a text data source 110, a natural language processor 120, an optional pseudonymization repository 130, and a pseudonymization processor 140. In the natural pseudonymization system 100, the text data source 110 either stores or receives text data. In some cases, the text data is structured data stored in a pre-defined data structure(s). The natural language processor 120 identifies sensitive text information in the text data. In some cases, the piece of sensitive text information is a piece of personal identifiable information. In some cases, the piece of sensitive text information is a piece of sensitive health information. In some cases, the natural language processor 120 identifies sensitive text information via a set of rules. In some cases, the natural language processor 120 identifies sensitive text information via data analysis and predictions, such as machine learning models, deep learning models, neural networks, and/or the like. The pseudonymization processor 140 may select natural pseudonyms and replace the sensitive text information identified by the natural language processor 120 with the selected natural pseudonyms.

A natural pseudonym refers to a piece of text data having at least one same information attribute as the piece of text data to be replaced. An information attribute refers to an attribute of the information contained in the text, for example, an information attribute can be a gender, an age, an ethnicity, an information type, a number of letters, a capitalization pattern, a geographic origin, street address characteristics of a location, and the like. For example, a piece of text data of "five" can be replaced with a natural pseudonym of "four" which has the same number of letters as "five". As another example, a piece of text data of "Nancy" can be replaced with a natural pseudonym of "Lisa" which is a name of the same gender as "Nancy".

In some cases, a natural pseudonym has at least two information attributes same as corresponding information attributes of the sensitive text information. In some embodiments, the natural pseudonym has a same number of letters as the piece of sensitive text information. In some cases, the sensitive text information is a person's name, and wherein the natural pseudonym is a person's name that is different from the sensitive text information. In some cases, the natural pseudonym enables a same downstream processing behavior as the sensitive text information. In some embodiments, the natural pseudonym cannot be distinguished from the sensitive text information by a reader. In some cases, the sensitive text information includes a first date range, and wherein the natural synonym has a second date range having the same duration as the first date range.

The text data source 110 can be any data repository storing text data including, for example, a software application transmitted text data, free text, structured document or database, a number of documents, a plurality of files, a relational database, a multidimensional database, object store system, and the like.

The natural language processor 120 can be implemented by one or more processors running machine readable computer instructions. In some embodiments, the text stream retrieved from the text data source 110 can be structured data, for example, data stored in a table with column name and data type identified. In such embodiments, the natural language processor 120 can identify the sensitive text information by the columns. For example, the column storing first name of the data structure contains sensitive text information. More details on classifying sensitive information is provided below.

In some cases, machine learning models are used and applied to estimate conditional probabilities of sensitive information types given terms, patterns, rules and contexts. In some example embodiments, different machine-learning models may be used. For example, bootstrapping context-based classifier, Bayesian statistical classification, logistic regression, naive-Bayes, random forest, neural networks, stochastic gradient descent, and support vector machines models may be used for classification. Additionally, deep learning models can be used, for example, convolutional neural network.

In some cases, machine learning bootstrapping is employed to estimate the sensitive information type and subtype of each term position in a document collection based on a previous iteration's best consensus estimate of the term's sensitive information type and subtype, and the current iteration's conditional probabilities are based on that consensus estimate. Human review and correction of learned/hypothesized sensitive information types and subtypes of terms is employed and utilized as further improved basis for the estimation of term sensitive information types and subtypes in subsequent iterations, as are periodically entered manual sensitive information type/subtype labels for new terms. The bootstrapping process is grounded by large imported, corrected and augmented datasets of sensitive information type and subtype labels, such as from census data.

The machine learning models can use framework of Bayesian statistical classification, and techniques for iteratively bootstrapping context-based classifiers from the observed contexts of seed instances, and then learning further specific type instances based on observation in a likely context of that type, using techniques such as the Expectation-Maximization (EM) algorithm, describe in more details in Dempster et al., "Maximum Likelihood from Incomplete Data via the EM Algorithm", Journal of the Royal Statistical Society, Series B. 39 (1): 1-38, 1977. The statistical text classification based on wide-context and narrow-context models can use machine learning models for discrimination in high dimensional spaces or use a combination of diverse features for lexical ambiguity resolution (Yarowsky, 1994), including multi-feature/multi-view bootstrapping learning methodologies such as the Yarowsky Algorithm (Yarowsky, 1995; Abney, 2004). The machine learning models and techniques are describe in more details in Gale, William A., Kenneth W. Church, and David Yarowsky. "Discrimination decisions for 100,000-dimensional spaces." Annals of Operations Research 55.2 (1995): 323-344; Yarowsky, David. "Decision lists for lexical ambiguity resolution: Application to accent restoration in Spanish and French." Proceedings of the 32nd annual meeting on Association for Computational Linguistics. Association for Computational Linguistics, 1994; Yarowsky, David. "Unsupervised word sense disambiguation rivaling supervised methods." 33rd annual meeting of the association for computational linguistics, 1995; Abney, Steven. "Understanding the Yarowsky algorithm." Computational Linguistics 30.3 (2004): 365-395.

In some cases, the pseudonymization processor 140 uses the pseudonymization repository 130 to select natural pseudonyms based on the identified sensitive text information. Natural pseudonyms are based on like-for-like replacements of fine-grained sensitive information types (e.g. female first names replaced by female first names, and Flemish street names replaced by Flemish street names). Additional constraints improve naturalness and minimize impact on downstream tasks (including via replacement of terms of equal length and replacement of like-for-like replacement of finer-grained term properties such as name ethnicity). The candidate replacement set for each sensitive information data type (and subtype) is a curated list stored in a database. Natural pseudonym replacement (e.g. Helga Schmidt for Gerda Mueller) is consistent within a defined replacement region which is optionally (1) a single document, (2) a single encounter (set of documents), (3) a complete patient record, (4) a batch of documents, (5) a particular document source or (6) the entire document database, and can optionally have a randomized different replacement value at each instance of a term.

Natural pseudonyms for numbers and identifiers preserve characteristic aspects of the identified sensitive information subtype, including length and numeric range, and such optional properties as replacing identifiers with naturally equivalent internal format (e.g. WBQ391T replaced by FNR147M).

In some cases, dates are shifted by a fixed offset "o" (e.g. in the range -180 to +180 days), preserving relative order of events, length of hospital stay, and duration relative to accident or admission. Dates more than one year previous are shifted by the same offset with an additional quantity of noise +/"n" (e.g. replacement date = original date +o +n), where the range of random variation of n increases proportionally to distance from the current date.

In some cases, the system 100 includes a pseudonym table 145 for each set of processed text data or documents. The pseudonym table 145 includes the mapping of the sensitive information and the corresponding natural pseudonym. The natural pseudonymization system 100 may provide the processed text data to a downstream processor 150. In some cases, the processed text data preserves the data processing results by the downstream processor 150. In some cases, the processed text data preserves the data processing behavior by the downstream processor 150. For example, a sequence of medical treatments can be properly coded. In some cases, the processed text data preserves byte offset relative to the beginning of the document.

In some embodiments, the natural pseudonymization system 100 may provide the pseudonymization information (e.g., pseudonym table 145) to a re-identification processor 160 to re-identify the pseudonymized downstream-processed text data with original sensitive information. For example, a female replaced first name is changed back to the actual female first name. In some cases, the downstream processor 150 may also provide downstream output to the re-identification processor 160. In some cases, the re-identification processor 160 may be a processor reside in the data controller environment as the natural pseudonymization system 100. In some cases, the re-identification processor 160 can re-identify the data based on pseudonym table. In some cases, the re-identification processor 160 can re-identify certain preselected fields (e.g., only location fields) or types of sensitive text data, but not the entire downstream-processed text data. In some cases, the re-identification processor 160 can re-identify the entire downstream-processed text data.

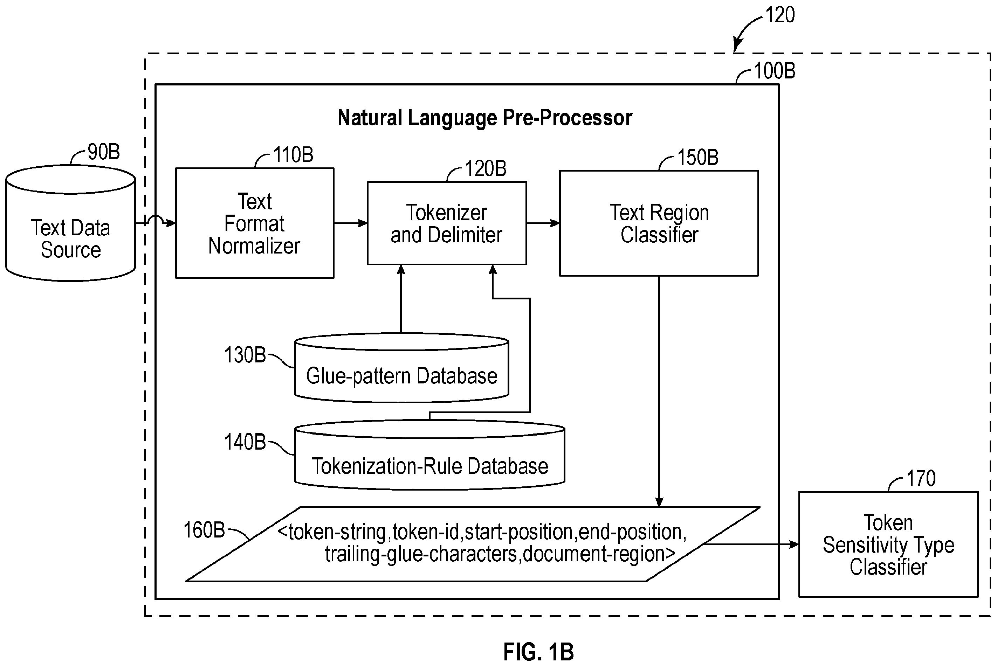

Figures 1B-1E provide some example implementations of components illustrated in Figure 1A. Figure 1B illustrates one example of natural language pre-processor 100B, which can be a component of the natural language processor 120. As illustrated, the natural language pre-processor 100B includes a text format normalizer 110B, a tokenizer and delimiter 120B, a glue-pattern database 130B, a tokenization-rule database 140B, a text region classifier 150B, and a token generator 160B, where each component is optional to the natural language pre-processor 100B. The natural language pre-processor 110B receives a text input from the text data source 110B and provides the generated tokens to token sensitive type classifier 170. The text format normalizer 110B is configured to normalize text to be processed by a tokenizer. For example, normalize a text into XML ("Extended Markup Language") formatted text or HTML ("Hyper Text Markup Language"). In one example, the tokenizer and delimiter 120B creates one token for each word in the text input with position number(s) and a glue for each special character. As used herein, a glue refers to the combined intervening material between content bearing tokens, including primarily punctuation, white space, line breaks and, in some embodiments, HTML/XML/RTF markup that convey formatting information rather than semantic content. In some embodiments, the glue-pattern database 130B is used to identify special characters, such as space, comma, new lines, and punctuation characters in multiple languages or the like, combined in some embodiments with patterns that recognize intervening HTML/XML/RTF markup spans that convey formatting information rather than semantic content. In some cases, the tokenizer and delimiter 120B creates a position number for each glue span (the combined set of glue characters between the current and previous semantic-content-bearing token. In some cases, each glue has a same position number as the word proceeding it. In general, the original content of the document can be reconstituted by alternating printing of semantic tokens and glue, and the new construction of deidentified documents are realized by the alternating printing of the pseudonymized content bearing tokens and the intervening, formatting-focused, non-content-bearing glue.

In some cases, the tokenizer and delimiter 120B uses the tokenization rule database 140B to create the tokens. For example, a number interpreted using the tokenization rule database 140B, such that the number series of 16.92 and 16,92 is created with a same token for text in different language. The text region classifier 150B identifies the text region in the context of a document for each word, such as footer, header, sections of documents, etc. The token generator 160B generates tokens for the text input. One example token is shown in 160B, <token-string, token-id, start-position, end-position, trailing-glue-characters, document-region>.

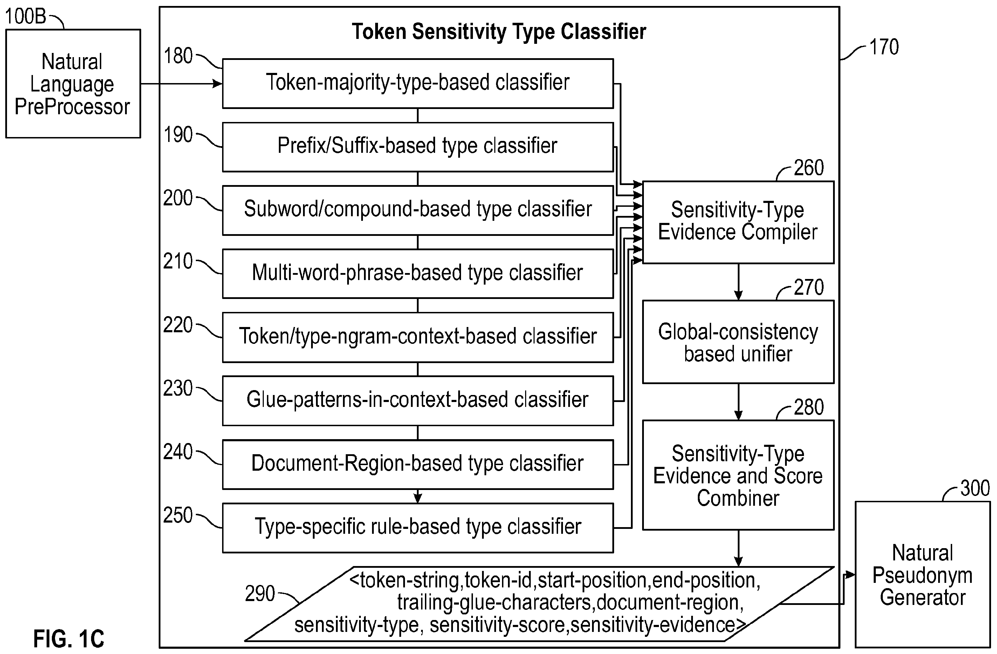

Figure 1C illustrates one example of the token sensitivity type classifier 170, which is another component of the natural language processor 120. In the example illustrated, the token sensitive type classifier 170 may use one or more of the following classifiers: a token-majority-type-based classifier 180, a prefix-suffix-based type classifier 190, a subword-compound-based type classifier 200, a multi-word-phrase-based type classifier 210, token/type-ngram-context based classifier 220, a glue-patterns-in-context-based classifier 230, a document-region-based type classifier 240, and a type-specific rule-based type classifier 250. Any one of the classifiers may use one or more machine learning models and deep learning models described above.

In one example, the token-majority-type-based classifiers 180 provide a baseline classification of each token based on the majority token sensitivity type of the token in the target language and domain. In some embodiments, for example, the baseline majority type of Wilson is LNAME (last name), Susan is FNAME(first-name)-FEMALE, and 'catheter' has majority category GENMED (General medical term). In some cases the majority category is language or domain sensitive; for example, in the configuration targeted at English clinical text the token MIT would have a baseline majority category such as INSTITUTION, while in German the same token would have a majority category as GENERAL (as a generic function word meaning 'with' in English). In each case, the confidence or strength of each classification is also represented as a score based on the prior/majority probability of that majority classification.

In one example, the prefix/suffix-based type classifiers 190 predict the majority type classification based for words not already assigned a majority type in the lexicon, based on variable-length word prefixes and suffixes. In some embodiments, for example, the suffixes '-litis', '-itis', '-tis' and '-is' in English clinical configuration all indicate a majority semantic category of GENMED (general-medical) for a word not currently in the lexicon such as 'asecolitis', and these scores are combined through weighted addition-based averaging. All relevant prefix/suffix indicators are combined for robustness, not only the longest matching one.

In one example, the subword/compound-based type classifiers 200 provide a complementary basis for assigning an initial majority type classification for tokens not already assigned a majority type in the lexicon, based on identifying existing in-lexicon tokens as subwords that collectively (or partially) combine to form the out-of-lexicon token, which is especially important for long compositional terminology such as found in German. In some embodiments, for example, the German token 'Phosphatbindertherapie' can be decomposed into in-lexicon entries 'Phosphat' 'binder' and 'therapie', which individually and in combination indicate (for example, in the medical domain) a GENMED (general-medical) majority semantic type. Intervening compounding characters (e.g. 's' between some compound-word components in German) and partial coverage are all allowed, with combined confidence based on the individual component type confidences, their type agreement, and their percentage of complete coverage.

In one example, the multi-word-phrase-based type classifiers 210 perform efficient hash-based lookup of multi-word-phrase-based patterns and their corresponding types, assigning type probabilities to multi-word expressions equivalently to single-word terms (such as the medical phrase 'Foley_catheter' to the type class GENMED (general-medical term in the medical domain).

In one example, the token/type-ngram-context-based classifiers 220 use surrounding n-gram context patterns to contribute component type probabilities based, in some embodiments, on variable-length word-ngram and class-ngram patterns of left, right and surrounding context. For example, the left-context literal word ngram patterns '<inserted><a>* => GENMED/score' or '<dear><dr>* => LNAME/score' may contribute component class prediction scores based on literal contexts, while ngram class patterns such as '[TITLE]*[LNAME] => FNAME' contribute component class prediction scores based on left and right word class contexts, based either on the static majority class (e.g. Wilson=>LNAME) or the current dynamic majority class. Mixed literal and type patterns such as '<mr>[FNAME]* => LNAME' and additional constraints current token-to-be-classified (e.g. '*' constrained to be numeric, etc.) are also supported and contributory.

In some embodiments, glue-patterns-in-context-based classifiers 230 contribute component class prediction scores based on surrounding glue patterns in context (e.g. "*<:>" vs. "<:>*") as per classifiers 180-220.

In some embodiments, document-region-based-type classifiers 240 contribute component class predictions scores favoring or deprecating the probability of type classes based on current document region classifications (e.g. a salutation or physical exam region) or based on densities of particular word types in broader context.

In some embodiments, the type-specific-rule-based type classifiers (250) support specialized classification patterns specific to the target word class in question (such as address components, dates, times, phone numbers or measure expressions) based on specialized phrasal grammars or token-internal syntactic patterns.

In some embodiments, the token sensitive type classifier 170 may further include one or more of a sensitivity-type evidence complier 260, a global-consistency based unifier 270, and a sensitivity-type evidence and score combiner 280. In one example, the sensitivity-type evidence complier 260 is configured to compile the evidence of sensitive-type, for example, the text region of the word. The evidence compilation includes the weighted combination of numeric scores based on the relative efficacy of each component, with negative scores (and a combined negative score combination) indicating sensitive terminology warranting pseudonymization and positive scores (and combined positive score combination) indicating non-sensitive terminology. Distinct from the core decision of sensitive/not-sensitive (psudonymize vs. not-pseudonymize) based on the combined consensus of the output of classifiers 180-250, a secondary decision is, within the core pseudonymize/not-pseudonymize classification, which class subtype (e.g. LNAME, FNAME, STREET, etc.) is individually the most probable, to trigger the appropriate natural pseudonym generation, analogously to weighted core-decision score combination but specific to each candidate data type, with the greatest absolute value score triggering the pseudonymization type. In one example, the global-consistency based unifier 270 is configured to designate one score to a word across multiple appearances in the text input. For example, the global-consistency based unifier 270 determines a sensitivity-score for a specific word by averaging sensitivity-scores of the word at various appearances. In one example, the sensitivity-type evidence and score combiner 280 compiles the sensitivity-type evidence gathered by the sensitivity-type evidence compiler 260 and compile a composite sensitivity score.

One example output of the token sensitivity type classifier 270 is illustrated as 290, <token-string, token-id, start-position, end-position, trailing-glue-characters, document-region, sensitivity-type, sensitivity-score, sensitivity-evidence>. The sensitivity-type indicates the type of sensitive information, such as name, address, and the like. The sensitivity-score indicates the probability of the word being sensitive information. In one example, a negative score represents a more than 50% likelihood of sensitive information. For clarity, the sensitivity-score does not indicate the probability of the word being a specific type of sensitive information, for example, a name. As one example, a score for "Madison" indicates how likely the word "Madison" is a piece of sensitive information, which can be a name, a place, a hospital name, etc.; however, the score does not indicate how likely the word "Madison" is a name.

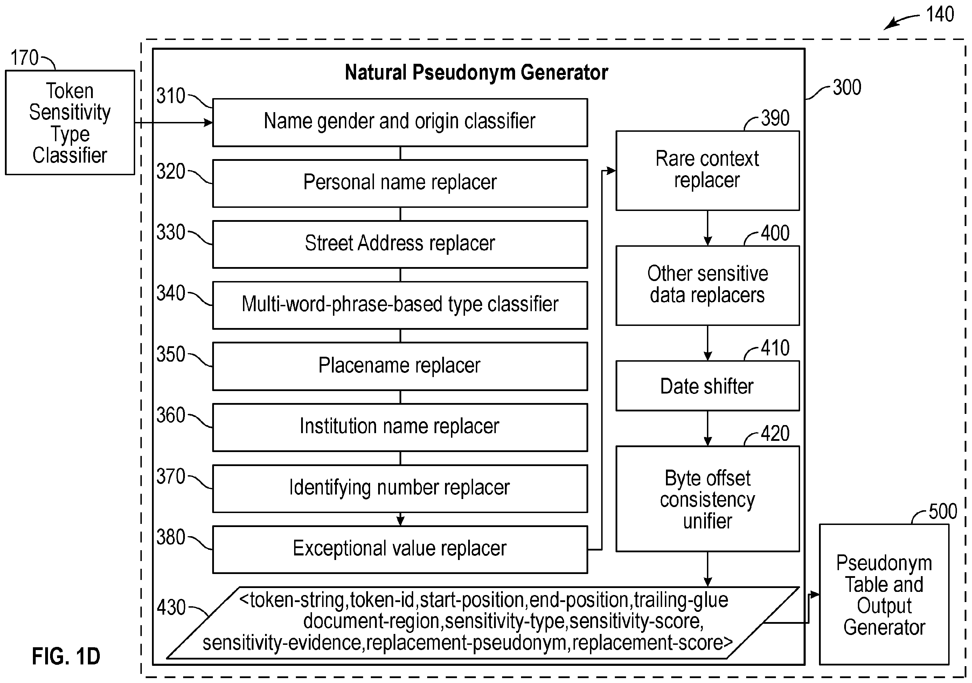

In some embodiments, the generated tokens by the token sensitivity type classifier 170 is sent to natural pseudonym generator 300. Figure 1D illustrates one example embodiment of a natural pseudonym generator 300, which is one component of the pseudonymization processor 140. In some embodiments, the natural pseudonym generator 300 includes one or more of a name gender and original classifier 310, a personal name replacer 320, a street address replacer 330, a multi-word-phrase-based type classifier 340, a placename replacer 350, an institution name replacer 360, an identifying number replacer 370, an exceptional value replacer 380, rare context replacer 390, other sensitive data replacer 400, data shifter 410, and byte offset consistency unifier 420. In some embodiments, the name gender and origin classifier (module 310) identifies the likely human gender and national/linguistic origin of given names (e.g. FNAME) and surnames (e.g. LNAME) previously classified as such via module 170. The evidence for this classification may include conditional probabilities over such features as variable-length name prefixes and suffixes, phrasal word order and context, and unigram prior probabilities as stored in the lexicon.

In one example, the name gender and original classifier 310 generates a classification code of the type of name to be replaced in a natural pseudonymization. For example, in conjunction with other available unigram and prior lexicon evidence, the name gender and original classifier 310 assigns a classification code for an out-of-lexicon name ending in the variable length affixes "-a", "-ina", etc. (such as 'Anadina') to indicate a female name classification code, with analogous classification features optionally employed for name linguistic/national origin.

Next, the name gender and national/linguistic origin classifier 310 provides the classification code(s) to the personal name replacer 320 as an input. The Personal Name replacer (320) selects a replacement natural pseudonym with database-stored characteristics compatible with the original name's code for gender, national/linguistic origin, number of characters, capitalization patterns and/or other such properties as set by the system configuration. In one example, a natural pseudonym is selected by the personal name replacer 320 for a name, referred to as SI-N, to be a name with the same gender and original classification (e.g., German female name) as the SI-N. In one example, a natural pseudonym is selected by the personal name replacer 320 for SI-N to be a name with the same number of characters.

In some embodiments, the Street Address replacer (330) selects a street address pseudonym for the original street address classified as such in module 170 (referred to as SI-A), with compatible characteristics to that original street address, including such properties as national, regional or linguistic origin, number/address format, number of characters, capitalization patterns, building number and unit number representations for the target region, and/or actual street names from the target region as pseudonym replacements.

In some embodiments, the multi-word-phrase-based type classifier and replacer (module 340) identifies other sensitive multi-word expressions (SI-M), optionally but not restricted to such address subcomponents as institutional department or subaddress, and their relevant type codes analogous to the processes in module 310-330, and generates a natural pseudonym replacement including the methods employed in modules 310-330

In some embodiments, and utilizing the processes employed in 310-330, the placename replacer 350 classifies the national and regional city, region, state, country and/or postcode properties and syntax for a placename identified as such in module 170, and selects replacement pseudonyms for the city, region, state, country and/or postcodes with consistent properties (national/regional/linguistic origin and address syntax) either from a database or artificially generated consistent, as in one example, with the legal syntax and numeric range of a region's postcodes/zipcodes. In one example, a placename replacer 350 is configured to select a replacer for a city, referred to as SI-C, with a city and optionally with a corresponding state and postcode/zip code. In one embodiment, a replacement city pseudonym is selected first and a randomly-chosen actual state/region and postcode/zip-code corresponding to that chosen city pseudonym is selected from a database of such actual city/region-state/postcode pairings for naturalness. In one embodiment, artificial new pseudonym placenames are generated using the natural multi-word phrasal ngram syntax of the region (e.g. "Winnekta Springs" replacing "Posselta Valley")

In one embodiment, the Institution Name Replacer 360 assigns codes for the institutional types and characteristics, including national/regional/linguistic origin, name syntax and word length, facility subtypes (e.g. hospital or radiology clinic or government/legal office), via database lookup, prefix/suffix ngrams and phrasal ngrams, and selects from a database and/or artificially generates institutional names with compatible properties consistent with the target configurations, as in modules 310-350.

In one embodiment, the Identifying Number Replacer (module 370) classifies the subtype and syntax of identifying numbers or alpha-numeric identifiers (referred to as SI-I), including length and numeric range, capitalization patterns, and/or legal placement and alternation of letters and numbers in the identifiers and any domain-specific properties (such as for a particular hospital's test ID or patient ID syntax) and generates natural artificial pseudonyms for those identifying numbers or alpha-numeric identifiers compatible with those properties.

In one embodiment, the Exceptional Value Replacer identifies those measure values (e.g. heights, weights), ages, birthdates, etc. that are in a configuration-specified range of values that are rare enough to be potentially identifying, especially in conjunction with other information (e.g. ages over 90). As per configuration, such exceptional values may be either replaced with a random numeric value replacement within the configuration-specified normal ranges, or replaced with a random replacement value within an acceptable replacement range consistent with the general trends/properties of the original value (e.g. old age or very high weight), but from a larger range so as to not be identifying within configured levels of risk.

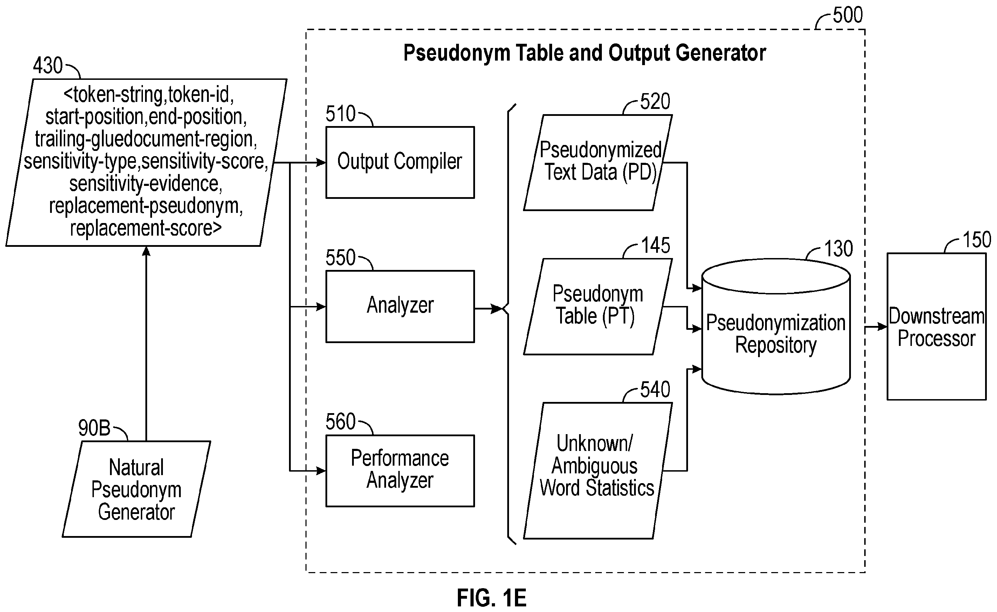

One example output of the natural pseudonym generator 300 is illustrated in 430, <token-string, token-id, start-position, end-position, trailing-glue-characters, document-region, sensitivity-type, sensitivity-score, sensitivity-evidence>. In some embodiments, such natural pseudonyms output 430 from the natural pseudonym generator 300 are provided to the pseudonym table and output generator 500, which is another component of the pseudonymization processor 140, with one example illustrated in more details in Figure 1E. In one embodiment, the pseudonym table and output generator 500 includes one or more of an output compiler 510, an input analyzer 550, and a performance analyzer 560. In some embodiments, the output complier 510 takes the natural pseudonyms 430 and the input text data to generate the pseudonymized text data (PD) 520 and optional pseudonym table (PT) 145. In one embodiment, each text input has an identification number (ID) and each PT is assigned with the same ID. In some implementations, the pseudonym table and output generator 500 saves PT to the pseudonymization repository 130.

In addition to generating pseudonymized documents (520) and the pseudonym tables 145 necessary for their re-identification, in some embodiments additional useful output may be produced. These include Unknown/Ambiguous Word Statistics for Product Improvement (540), which include exports of specific terms observed in documents processed by the system (in training or production modes) which are not currently in the classification databases, along with the system's best estimation of their sensitive information type based on the context patterns used for classification in module 170 (e.g. a likely first name identified based on its occurrence between a title (e.g. Dr.) and known/likely surname (e.g. "<Dr>*[LNAME] => FNAME/score), and these predictive context-based type classifications above a certain confidence threshold (in isolation or based on combination of multiple independent predictions at weaker confidence) can be aggregated and added to the unigram majority class tables (with the predicted confidences) for future encounters of that term without adequately identifying context. Likewise, terms with multiple sensitive information types may have exported frequency counts of the aggregated number of instances of each sensitive information type predicted based on context, so an improved prior probability distribution (and term/type/confidence conditional probability scores) can be better estimated.

In some embodiments, full input analysis data structures are generated, including annotations of the original documents, predicted types, confidences, and replacements, such that anomalous behavior can be debugged and/or so that context patterns can be aggregated and improved.

In some embodiments, analysis performance statistics are generated so that system users and/or system developers can understand and/or further analyze the range of sensitive information types observed in documents from a particular source, so, for example, that the observed risk of documents from that source can be quantified and additional protection protocols implemented.

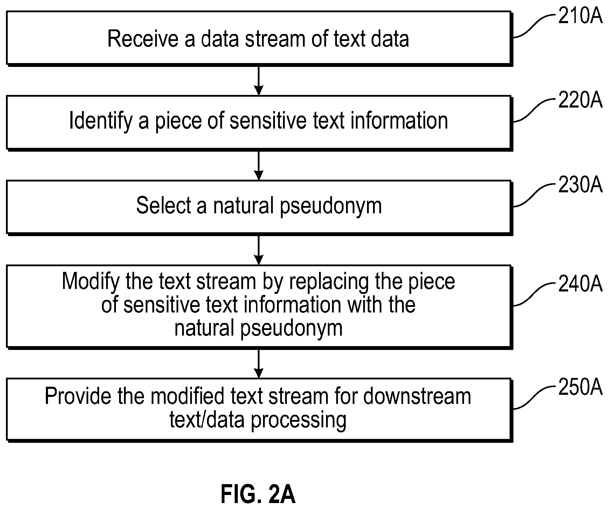

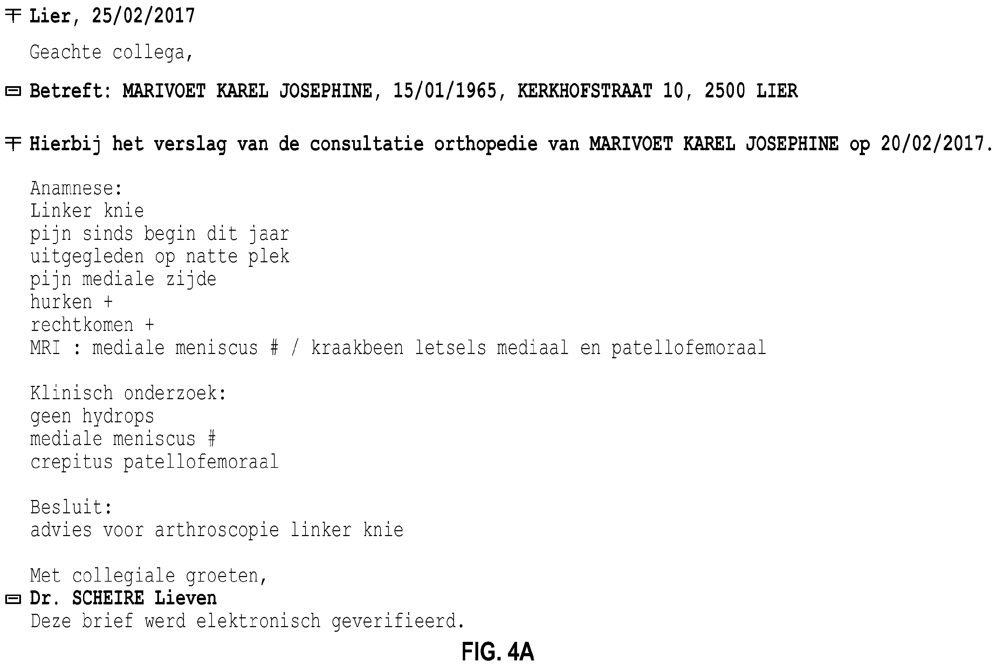

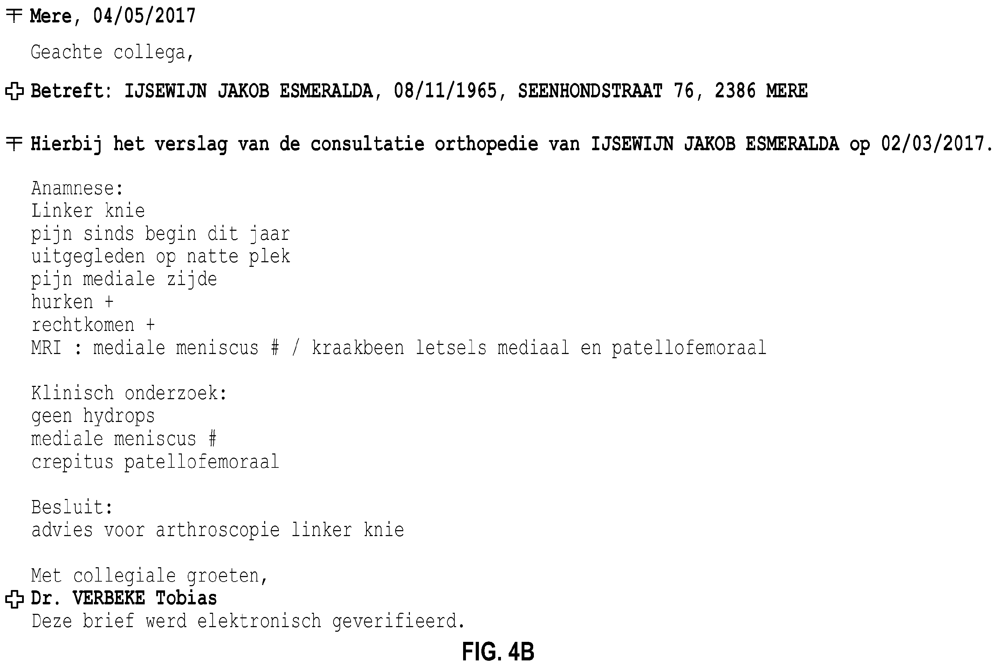

Figure 4A and 4B illustrate one example of input and output of a natural pseudonymization system. In some cases, a natural pseudonym method and system can improve the performance of downstream processing. In some cases, the output of a natural pseudonym method and system can allow a human reviewer or a machine reviewer to review a document that is pseudonymized in an ordinary way. Figure 2A illustrates a flowchart of one embodiment of a natural pseudonymization system. The system receives a data stream of text data (step 210A). In some cases, the system retrieves the data stream from a data source. Next, the system identifies a piece of sensitive text information in the data stream (step 220A). In some cases, the system identifies the sensitive text information using the data structure.

**In** one embodiment, the following are selected and partial examples of the kinds of machine learning features employed in modules 180-400 for the classification of sensitive information types and subtypes.:
(1) term unigram scores for P(sensitive information-class|keyterm) - probability, keyterm is the original text data, for each term
(2) term ngram probabilities for P(sensitive information-class|ngram) including term ngrams of multiple lengths surrounding keyterm
(3) class ngram probabilities for P(sensitive information-class|class-ngram) including sensitive information-class ngrams of multiple lengths surrounding keyterm, such as P(sensitive information-class=FNAME|<position-1=TITLE><position+1=LNAME)
(4) affix probabilities for P(sensitive information-class|affix) including term prefixes and suffixes of multiple lengths (e.g. P(GENMED|<suffix5="itis")'
(5) affix probabilities for P(sensitive information-class|affix) including term prefixes and suffixes of multiple lengths
(6) region probabilities for P(sensitive information-class|region) for the current wide context region (e.g. P(GENMED|<region=physical_exam>)
(7) glue context probabilities for P(sensitive information-class|glue-context) for multiple components of glue context (e.g. P(sensitive informationclass=TITLE|<glue+1=".,">))
(8) developer-defined rule probabilities for P(sensitive information-class|rule-triggers) for multiple developer defined rule triggers (e.g. email address patterns)
(9) specialized rule probabilities for numbers and other specialized sensitive information types, including numeric ranges and subcomponents consistent with sensitive information types such as postal codes or phone numbers.

In one example, sensitive information types and subtypes can be predetermined and constitute a grammar of substitutable equivalence classes. For example, the following sample address (SI-Example):
Prof. Helga Schmidt
37 Eigerstr
41460 NEUSS
corresponds to the sensitive information subtypes:
TITLE FNAME LNAME
ADDRNUM STREET
POSTCODE CITY

An example of corresponding natural pseudonyms from the same equivalence classes for SI-Example is provided below:
Prof. Gerda Mueller
52 Hildeweg
47441 MOERS

In one example, sensitive information subtypes can be pre-defined, and the sensitive information subtype CITY can be further subclassified as LARGECITY or SMALLCITY if particular downstream applications, such as language modeling for speech recognition, require such precision. In some implementations, the substitution space is definable by preconfigured region of application (e.g. at the country level, where GERMAN cities/streets/names are replaced by other German cities/streets/names, or at a more precise regional level, where BELGIUM-FLEMISH cities/streets/names are replaced by other cities/streets/names. Further naturalness is achieved by generating postcodes that correspond to the randomly chosen city substitution (e.g. 47441 is a legal postcode in MOERS Germany). In some implementations, the capitalization formatting of the replacements is preserved (e.g. NEUSS in all caps is replaced by MOERS in all caps).

The system selects a natural pseudonym for the piece of sensitive text information (step 230A). The system modifies the text stream by replacing the piece of the sensitive text information with the natural pseudonym (step 240A). Optionally, the system provides the modified text stream for downstream data processing or text processing (step 250A).

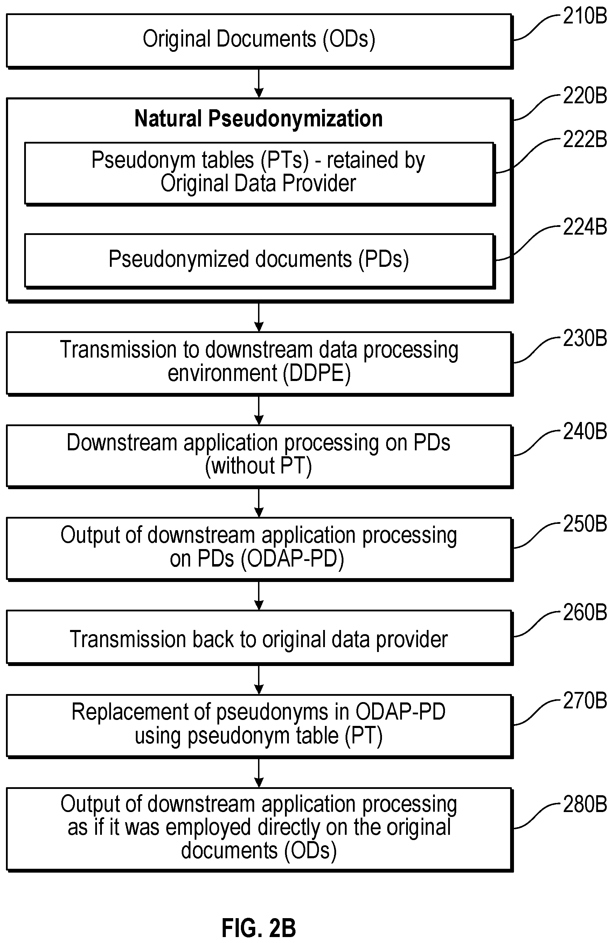

Figure 2B illustrates another example flowchart of a natural pseudonymization system working with a downstream processing system. One or more steps are optional in this flowchart. The system receives original documents (ODs) or retrieves ODs (step 210B). The system conducts natural pseudonymization using any of the embodiments described in the present disclosure (step 220B) to generate pseudonymized documents (PDs) (224B). In some cases, pseudonym tables (PTs) (222B) can be generated and retained by the original data provider. Next, the pseudonymized documents (PDs) are transmitted to downstream data processing environment (DDPE) (step 230B). Further, downstream processing system processes the PDs (without PT) (step 240B). Output of downstream application processing on PDs (ODAP-PD) are generated (step 250B). The outputs are transmitted back to original data provider (step 260B). In some cases, the pseudonyms are replaced in ODAP-PD using pseudonym table (step 270B). A re-identified downstream processing output is then generated (step 280B).

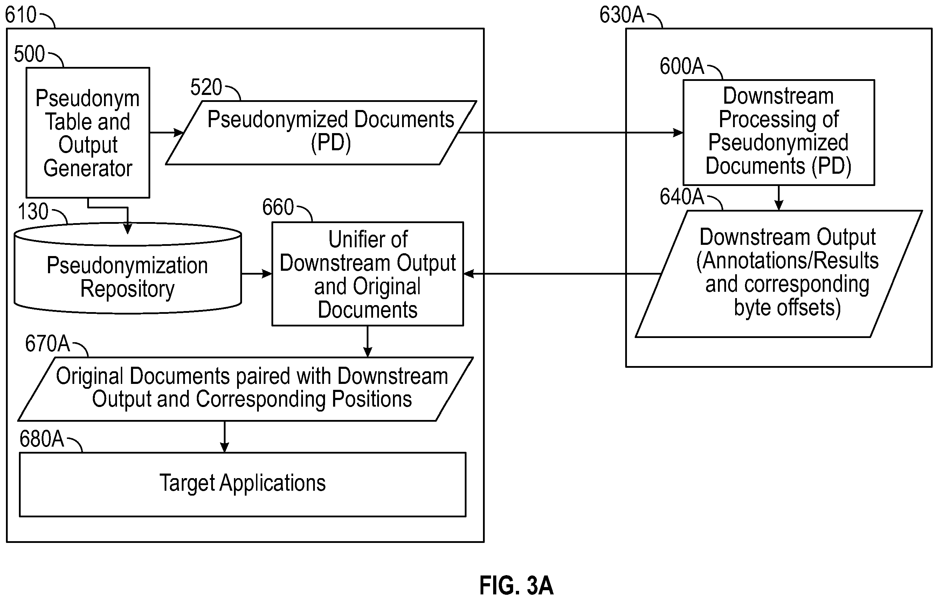

Figures 3A-3E illustrate various examples of how natural pseudonymization systems used with downstream processing systems. In the figures, components labeled with same numbers indicate same or similar components, including components described in Figures 1A-1E. Figure 3A illustrates one example data flow diagram of a natural pseudonymization system 610A working with a downstream processor 630A. The natural pseudonymization system 610A includes the pseudonym table and output generator 500, with on example implementation illustrated in Figure 1E and a unifier of downstream output and original documents 660. The pseudonym table and output generator 500 generates pseudonymization documents 520, where such documents 520 are transmitted to downstream processor 630A. In some cases, the generator 500 creates pseudonym table and stores it into the pseudonymization repository 130. In some cases, the original document and the pseudonymized document are all stored in the pseudonymization repository 130. The downstream processor 630A includes a processor 600A to provide desired processing of pseudonymized documents, for example, annotations, data processing, code generation using the documents, or the like. The processor 600A generates downstream outputs 640A. In one case, the downstream output 640A includes annotations of the pseudonymized documents. In one case, the downstream output 640A includes processing results of the pseudonymized documents. In an example, the downstream output 640A includes byte offsets information.

The downstream output 640A is transmitted back to the natural pseudonymization system 610A. The unifier 660 retrieves the original documents and combines it with the downstream output. In one example, the unifier 660 generates the document suite of original documents paired with downstream output 670A and provides the document suite to target applications 680A.

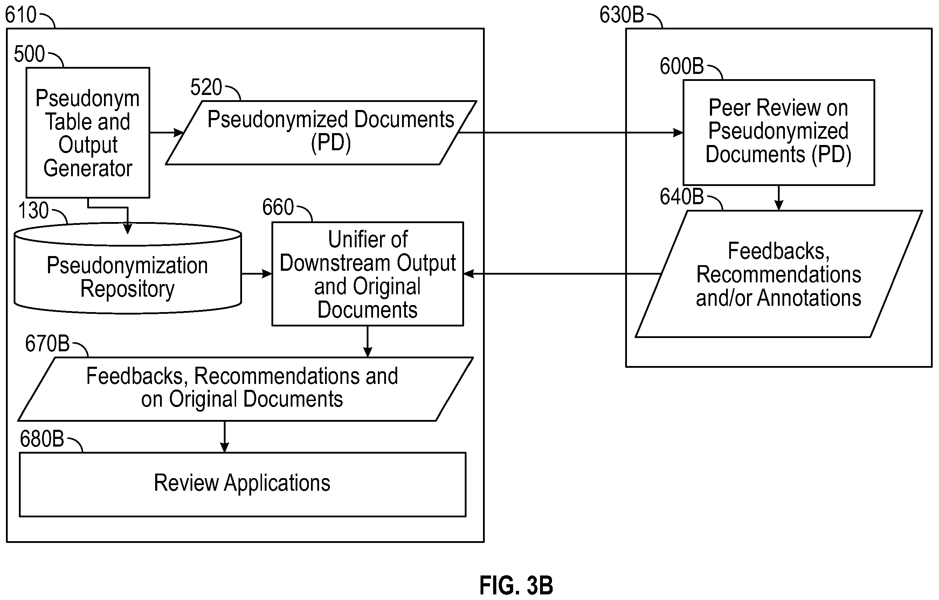

Figure 3B illustrates an example flow diagram of a natural pseudonymization system 610B used for peer review. In this example, the downstream processor 630B receives the pseudonymized documents and presented them to peer reviewers via a device 600B. The peer reviewers will provide downstream output 640B that is a reviewer report in this embodiment, for example, feedbacks, recommendations, annotations, or the like. The reviewer report 640B is transmitted back to the natural pseudonymization system 610B. The unifier 660 will merge the reviewer report 640B with the original documents and generates reviewer report (e.g., feedbacks, recommendations, and annotations) on original documents, referred to as re-identified reviewer report 670B. The re-identified reviewer report 670B is provided to targeted review applications 680B.

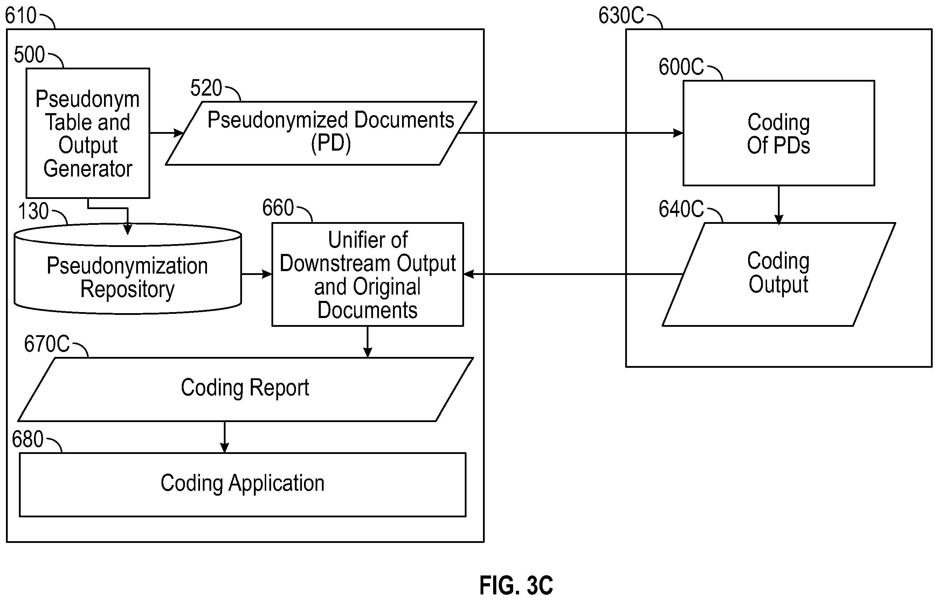

Figure 3C illustrates one other example flow diagram of a natural pseudonymization system 610C used for coding, which is to generate predetermined codes based on the information in documents. In this example, the downstream processor 630C receives the pseudonymized documents and ran them through a coding application 600C supporting manual, semi-automated, or automated coding. The coding application 600C generates coding output 640C, which may include codes and optionally evidence information, such as the location of the evidence within the pseudonymized documents. The coding output 640C is transmitted back to the natural pseudonymization system 610C. The unifier 660 may merge the coding output with the original documents to generate a coding report 670C. In one example, the coding report 670C includes assigned codes and evidence markup in the original documents. The coding report 670C is provided to coding application 680C, for example, for statistical report generations, audit, and payment processing.

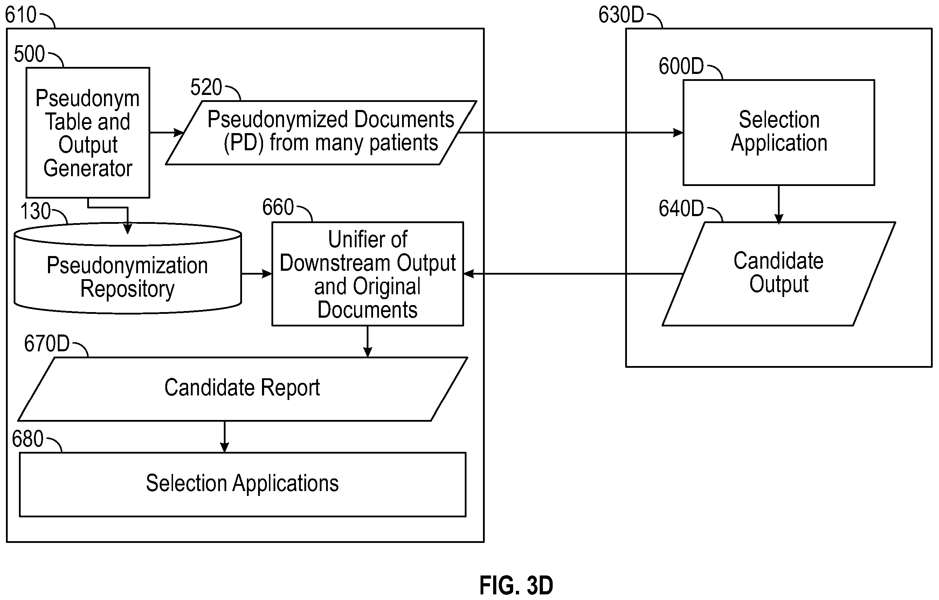

Figure 3D illustrates one other example flow diagram of a natural pseudonymization system 610D used for candidate selections. In this example, the downstream processor 630D receives the pseudonymized documents and ran them through a candidate selection application 600D supporting manual, semi-automated, or automated candidate selection. For this downstream application, it is important to keep information relevant to selection criteria to be the same, such as gender, age, medical history, social economy status, or the like. The candidate selection application 600D generates candidate output 640D, which may include each candidate's information. The candidate output 640D is transmitted back to the natural pseudonymization system 610D. The unifier 660 may merge the candidate output with the original documents to generate a candidate report 670D. In one example, the candidate report 670D includes actual names, identifications, contact information of the selected candidate(s). The candidate report 670D is provided to selection application or candidate repository 680D. In one example, the selection application 680D is configured to generate an output with minimal contact information of the candidate to make it available to the agents.

In one embodiment, pseudonymized document transmission to downstream data processors such as medical coding applications, case management applications, clinical statistics applications, information extraction applications, document abstracting and summarization applications, etc. where the sensitive information isn't necessary for the successful execution of these applications, and where the outcome of these applications is unaffected by the natural pseudonym replacement.

In some embodiments, the documents are stored at rest in pseudonymized form (PD), separately from their pseudonym tables (PT) for additional security even within the original data provider environment. If downstream application processing is unaffected by the OD to PD pseudonymization, then pseudonymized storage at rest separate from a carefully guarded pseudonym table PT may be a desirable standard storage mechanism for the primary working data.

In some cases, the downstream data processing involves human analysis and review, including manual human equivalents of the automated downstream data processing applications (such as manual medical coding). Manual human processing replaces the automated downstream data processing application in the flowchart. It is important for efficient of human processing that documents preserve natural structure and content (e.g. with a female patient name replacing a female patient name rather than replaced by a the same to interpret random identifier such as X391748).

In some cases, the downstream data processing involves flexible human analysis, review and communication, such as: peer review of medical cases seeking peer advice; expert/specialist consultation of difficult medical cases; and clinical study participant selection and recruitment. It is particularly important when humans are expected to review and discuss pseudonymized documents that they look and read like normal documents with natural replacements. For example, in peer review or consulting uses, the reviewing/consulting physicians will discuss the patient as if she was "Gerda Mueller" rather than her actual name "Helga Schmidt".

In some embodiments, an additional software application/service is provided that selectively reidentifies needed sensitive information in the PD, such as the actual names and phone numbers of selected clinical study participants. For example, limited logged authorized lookup of necessary sensitive information components of the PDs via a separate lookup application or interface (e.g. an app that returns "Helga Schmidt" for the pseudonym "Gerda Mueller").

In some implementations of a natural pseudonymization system, only a selected range of sensitive information are replaced with natural pseudonyms to create a SRPD (selectively reidentified pseudonymized document) set with necessary and authorized sensitive information fields reidentified in situ (and logged). In some cases, the selective sensitive information replacement is performed temporarily and virtually on sensitive information region click or mouseover, but a reidentified document is not generated for storage. In some cases, a selected subset of PDs are fully reidentified to their original state (OD) using the PT, with appropriate authorization and logging.

In some cases, a process for measuring the efficacy of deidentification/pseudonymization that replaces sensitive health information terms with naturally behaving pseudonyms that when substituted with the original sensitive health information terms yield the same medical coding software output, medical data analysis software output, and automated medical-decision-making software output as when such software is executed on the original medical document prior to the pseudonym substitutions.

The present invention should not be considered limited to the particular examples and embodiments described above, as such embodiments are described in detail to facilitate explanation of various aspects of the invention. Rather the present invention should be understood to cover all aspects of the invention, including various modifications, equivalent processes, and alternative devices falling within the scope of the invention as defined by the appended claims

## Claims

1. A computer system comprising:
one or more computer processors;
one or more computer readable memories in communication with the one or more computer processors and having instructions stored thereon that, when executed by the one or more computer processors, cause the one or more processors to perform operations comprising
receive a data stream of processed text data;
reidentify (270B), by the one or more processors, a piece of sensitive text information in the processed text data using a pseudonym table (145; 222B), wherein the pseudonym table (145; 222B) contains a mapping of the piece of sensitive text information with a natural pseudonym,
wherein the natural pseudonym has at least one information attribute that is the same as a corresponding information attribute of the piece of sensitive text information such that the natural pseudonym is difficult to distinguish from the sensitive text information in the data stream,
wherein the reidentifying step (270B) comprises modifying the processed text data by replacing the natural pseudonym with the piece of sensitive text information,
**characterised in that** the sensitive text information includes a first date range,
wherein the natural pseudonym has a second date range having a same duration as the first date range.

2. The computer system of claim 1, wherein the at least one information attribute comprises at least one of a gender, an age, an ethnicity, an information type, a number of letters, a capitalization pattern, a geographic origin, and street address characteristics of a location.

3. The computer system of claim 1 or 2, wherein the natural pseudonym has at least two information attributes that are the same as corresponding information attributes of the piece of sensitive text information.

4. The computer system of any one of claims 1-3, wherein the piece of sensitive text information is a piece of personal identifiable information.

5. The computer system of any one of claims 1-4, wherein the piece of sensitive text information is a piece of sensitive health information.

6. The computer system of any of claims 1-5, wherein the natural pseudonym has a same number of letters as the piece of sensitive text information.

7. The computer system of any of claims 1-6, wherein the sensitive text information is a person's name, and wherein the natural pseudonym is a person's name that is different from the sensitive text information.

8. The computer system of any of claims 1-7, wherein the natural pseudonym enables a same downstream processing behavior as the sensitive text information.

9. The computer system of any of claims 1-8, wherein the natural pseudonym cannot be distinguished from the sensitive text information by a reader.

## Patentansprüche

1. Ein Computersystem, aufweisend:
einen oder mehrere Computerprozessoren;
einen oder mehrere computerlesbare Speicher, die in Kommunikation mit dem einen oder den mehreren Computerprozessoren stehen und auf denen die Anweisungen gespeichert sind, die, wenn sie durch den einen oder die mehreren Computerprozessoren ausgeführt werden, veranlassen, dass der eine oder die mehreren Prozessoren Operationen durchführen, aufweisend
Empfangen eines Datenstroms von verarbeiteten Textdaten;
erneut identifizieren (270B), durch den einen oder die mehreren Prozessoren, eines Teils sensibler Textinformationen in den verarbeiteten Textdaten unter Verwendung einer Pseudonymtabelle (145; 222B), wobei die Pseudonymtabelle (145; 222B) eine Zuordnung des Teils sensibler Textinformationen mit einem natürlichen Pseudonym enthält,
wobei das natürliche Pseudonym mindestens ein Informationsattribut aufweist, das dasselbe ist wie ein entsprechendes Informationsattribut des Teils sensibler Textinformationen, derart, dass das natürliche Pseudonym in dem Datenstrom schwer von den sensiblen Textinformationen zu unterscheiden ist,
wobei der Schritt des erneuten Identifizierens (270B) ein Modifizieren der verarbeiteten Textdaten durch Ersetzen des natürlichen Pseudonyms mit dem Teil sensibler Textinformationen aufweist,
**dadurch gekennzeichnet, dass** die sensiblen Textinformationen einen ersten Datumsbereich einschließen, wobei das natürliche Pseudonym einen zweiten Datumsbereich aufweist, der dieselbe Dauer aufweist wie der erste Datumsbereich.

2. Das Computersystem nach Anspruch 1, wobei das mindestens eine Informationsattribut mindestens eines von einem Geschlecht, einem Alter, einer Ethnizität, einem Informationstyp, einer Anzahl von Buchstaben, einem Großschreibungsmuster, einem geografischen Ursprung und Straßenadressenmerkmalen eines Standorts aufweist.

3. Das Computersystem nach Anspruch 1 oder 2, wobei das natürliche Pseudonym mindestens zwei Informationsattribute aufweist, die dieselben sind wie entsprechende Informationsattribute des Teils sensibler Textinformationen sind.

4. Das Computersystem nach einem der Ansprüche 1 bis 3, wobei der Teil sensibler Textinformationen ein Teil personenbezogener identifizierbarer Informationen ist.

5. Das Computersystem nach einem der Ansprüche 1 bis 4, wobei der Teil sensibler Textinformationen ein Teil sensibler Gesundheitsinformationen ist.

6. Das Computersystem nach einem der Ansprüche 1 bis 5, wobei das natürliche Pseudonym dieselbe Anzahl von Buchstaben aufweist wie der Teil sensibler Textinformationen.

7. Das Computersystem nach einem der Ansprüche 1 bis 6, wobei die sensiblen Textinformationen ein Name einer Person sind, und wobei das natürliche Pseudonym ein Name einer Person ist, der von den sensiblen Textinformationen verschieden ist.

8. Das Computersystem nach einem der Ansprüche 1 bis 7, wobei das natürliche Pseudonym ein gleiches nachgelagertes Verarbeitungsverhalten wie die sensiblen Textinformationen ermöglicht.

9. Das Computersystem nach einem der Ansprüche 1 bis 8, wobei das natürliche Pseudonym durch einen Leser nicht von den sensiblen Textinformationen unterschieden werden kann.

## Revendications

1. Système informatique, comprenant :
un ou plusieurs processeurs informatiques ;
une ou plusieurs mémoires lisibles par ordinateur en communication avec ledit un ou lesdits plusieurs processeurs informatiques et ayant des instructions stockées sur celles-ci qui, lorsqu'elles sont exécutées par ledit un ou lesdits plusieurs processeurs informatiques, amènent ledit un ou lesdits plusieurs processeurs informatiques à effectuer des opérations comprenant
la réception d'un flux de données de données textuelles traitées ;
la réidentification (270B), par ledit un ou lesdits plusieurs processeurs , d'un élément d'information textuelle sensible dans les données textuelles traitées à l'aide d'une table de pseudonymes (145 ; 222B), dans lequel la table de pseudonymes (145 ; 222B) contient une mise en correspondance de l'élément d'information textuelle sensible avec un pseudonyme naturel,
dans lequel le pseudonyme naturel a au moins un attribut d'information qui est identique à un attribut d'information correspondant de l'élément d'information textuelle sensible, de telle sorte que le pseudonyme naturel est difficile à distinguer des informations textuelles sensibles dans le flux de données,
dans lequel l'étape de réidentification (270B) comprend la modification des données textuelles traitées en remplaçant le pseudonyme naturel par l'élément d'information textuelle sensible,
**caractérisé en ce que** les informations textuelles sensibles comportent une première plage de dates, dans lequel le pseudonyme naturel a une seconde plage de dates ayant une même durée que la première plage de dates.

2. Système informatique selon la revendication 1, dans lequel l'au moins un attribut d'information comprend au moins l'un parmi un genre, un âge, une origine ethnique, un type d'information, un nombre de lettres, un motif de mise en majuscule, une origine géographique et des caractéristiques d'adresse postale d'un emplacement.

3. Système informatique selon la revendication 1 ou 2, dans lequel le pseudonyme naturel a au moins deux attributs d'information qui sont identiques à des attributs d'information correspondants de l'élément d'information textuelle sensible.

4. Système informatique selon l'une quelconque des revendications 1 à 3, dans lequel l'élément d'information textuelle sensible est un élément d'information personnelle identifiable.

5. Système informatique selon l'une quelconque des revendications 1 à 4, dans lequel l'élément d'information textuelle sensible est un élément d'information sensible de santé.

6. Système informatique selon l'une quelconque des revendications 1 à 5, dans lequel le pseudonyme naturel a le même nombre de lettres que l'élément d'information textuelle sensible.

7. Système informatique selon l'une quelconque des revendications 1 à 6, dans lequel les informations textuelles sensibles sont le nom d'une personne, et dans lequel le pseudonyme naturel est le nom d'une personne qui est différent des informations textuelles sensibles.

8. Système informatique selon l'une quelconque des revendications 1 à 7, dans lequel le pseudonyme naturel permet un même comportement de traitement en aval que les informations textuelles sensibles.

9. Système informatique selon l'une quelconque des revendications 1 à 8, dans lequel le pseudonyme naturel ne peut pas être distingué des informations textuelles sensibles par un lecteur.
